# EUROPÄISCHE PATENTSCHRIFT

(11) **EP 0 612 732 B1**
(45) Veröffentlichungstag und Bekanntmachung des Hinweises auf die Patenterteilung: **17.07.1996**
(21) Anmeldenummer: 94102318.6
(22) Anmeldetag: 16.02.1994
(51) Int. Cl.: C07D 235/16

(54) **Verfahren zur Herstellung von substituiertem und unsubstituiertem 2-(Cyanmethyl)benzimidazol**
Process for the production of (un)substituted 2-(cyanomethyl)benzimidazole
Procédé pour la préparation des 2-(cyanométhyle)benzimidazoles éventuellement substitués

(30) Priorität: 25.02.1993 DE 4305714
(43) Veröffentlichungstag der Anmeldung: 31.08.1994
(73) Patentinhaber: HOECHST AKTIENGESELLSCHAFT, 65929 Frankfurt am Main (DE)
(72) Erfinder: Dietz, Erwin, Dr., D-65779 Kelkheim/Ts (DE); Kapaun, Gustav, Dr., D-65812 Bad Soden (DE)

(56) Entgegenhaltungen:
- DE-A- 2 900 506
- FR-A- 1 361 778

## Beschreibung

2-(Cyanmethyl)benzimidazol ist eine bekannte Verbindung. Ihre Herstellung ist beispielsweise in DE-A-2 900 506, in der indischen Patentschrift 154 773, in FR-A-1 361 778 sowie in Sawlewicz, J. und Milczarska, B., Pol. J. Pharmacol. Pharm. 26, (1974), S. 642 beschrieben, wobei in den beiden letztgenannten Literaturstellen auch die Herstellung substituierter 2-(Cyanmethyl)benzimidazole beschrieben wird.

In den genannten Veröffentlichungen verläuft die Herstellung von 2-(Cyanmethyl)benzimidazol nach folgendem Schema: 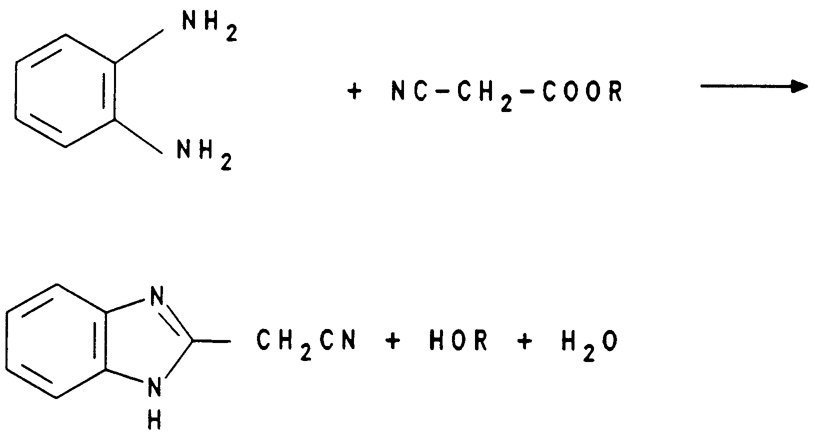
In der DE-A-2 900 506 erfolgt diese Kondensation ohne Verwendung eines Lösemittels. Die Nachteile bestehen darin, daß man verunreinigte Produkte und nur geringe Ausbeuten erhält.

In der FR-A-1 361 778 und in der indischen Patentschritt 154 773 werden halogenierte Aromaten, vor allem o-Dichlorbenzol, als Lösungsmittel eingesetzt.

Desweiteren wird der Einsatz eines sauren Katalysators beschrieben, vorzugsweise einer aromatischen Sulfonsäure.

In Sawlewicz, J. und Milczarska, B., Pol. J. Pharmacol. Pharm. 26, (1974), S. 642 wird 2-(Cyanmethyl)benzimidazol ohne Katalysator in wasserfreiem Xylol unter Rückfluß hergestellt. Die Ausbeute liegt jedoch nur bei 67 %.

Der vorliegenden Erfindung lag die Aufgabe zugrunde, ein Verfahren zur Herstellung von substituiertem und unsubstituiertem 2-(Cyanmethyl)benzimidazol zur Verfügung zu stellen, welches die Nachteile halogenierter Lösemittel, geringer Ausbeuten und verunreinigter Produkte überwindet.

Es wurde gefunden, daß man 2-(Cyanmethyl)benzimidazol und seine Derivate überraschenderweise auch ohne einen Katalysator in guten Ausbeuten und hoher Reinheit herstellen kann, indem man die Kondensationsreaktion bei einer Temperatur im Bereich von 150 bis 175 °C in einem halogenfreien inerten Lösemittel, dessen Siedepunkt gleich oder höher als 150 °C ist, oder in einer Mischung solcher Lösemittel durchführt.

Gegenstand der Erfindung ist ein Verfahren zur Herstellung von substituiertem oder unsubstituiertem 2-(Cyanmethyl)benzimidazol der Formel (I) 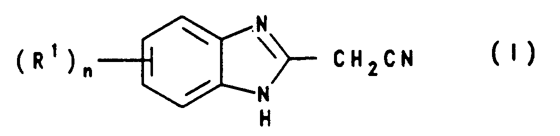
dadurch gekennzeichnet, daß man ein o-Phenylendiamin der Formel (II) 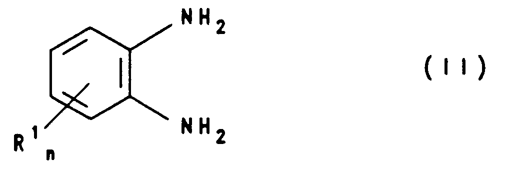
wobei R¹ C₁-C₄-Alkyl oder C₁-C₄-Alkoxy, vorzugsweise Methyl oder Methoxy, bedeutet und n eine ganze Zahl von 0 bis 2, vorzugsweise 0 oder 1, insbesondere 0, ist,
mit einem Cyanessigsäureester der Formel (III)

NC-CH₂-COOR² (III)

wobei R² lineares, verzweigtes oder cyclisches C₂-C₁₂-Alkyl bedeutet und 1 bis 3 CH₂-Gruppen des Alkylrestes jeweils durch Sauerstoff ersetzt sein können, bei einer Temperatur von 150 - 175 °C in einem halogenfreien, in Bezug auf die Reaktanden unter den gewählten Reaktionsbedingungen inerten Lösemittel, das einen Siedepunkt gleich oder höher als 150°C hat und aus der Gruppe der Cycloaliphaten, Olefine, Cycloolefine, Aromaten oder Araliphaten ausgewählt ist, wobei besagte Lösemittel auch Sauerstoffatome enthalten können, oder in einem Gemisch aus zwei oder mehreren solcher Lösemittel, umsetzt, ohne eine aromatische Sulfonsäure als Katalysator zuzusetzen.

Als o-Phenylendiamine sind beispielsweise von Interesse: 4-Methyl-1,2-diaminobenzol, 4-Methoxy-1,2-diaminobenzol und 4-Ethoxy-1,2-diaminobenzol. Besonders bevorzugt ist unsubstituiertes 1,2-Diaminobenzol.

Als Cyanessigsäureester sind diejenigen von Interesse, deren Rest R² Ethyl, n-Propyl, n-Butyl, iso-Butyl, n- und iso-Pentyl, 2-Ethylhexyl, n-Octyl, 2-Methoxyethyl oder 2-Ethoxyethyl ist. Bevorzugt sind die n-Propylester, n- und iso-Butylester und -Pentylester der Cyanessigsäure, besonders bevorzugt Cyanessigsäure-n-butylester und Cyanessigsäure-iso-butylester.

Die Cyanessigsäureester der Formel (III) können einzeln oder als Mischung eingesetzt werden. Insbesondere bei der Verwendung von Cyanessigsäureethylester ist es vorteilhaft, mindestens einen weiteren Cyanessigsäureester der Formel (III) dem Reaktionsgemisch zuzusetzen. Der Einsatz von Cyanessigsäureethylester allein ist grundsätzlich zwar auch möglich, aber das 2-(Cyanmethyl)benzimidazol fällt in schlecht kristalliner Form an, was zu Schwierigkeiten bei der Aufarbeitung führt. Diesen Nachteil kann man durch Zusatz von anderen Cyanessigsäurealkylestern, beispielsweise von Cyanessigsäure-n- oder -isobutylester überwinden. Besonders bevorzugt ist daher ein Gemisch aus Cyanessigsäureethylester mit Cyanessigsäure-n-butylester oder mit Cyanessigsäure-iso-butylester.

Man kann ein Gemisch aus Cyanessigsäureethylester und -n-butylester erhalten, indem man Cyanessigsäureethylester mit n-Butanol umestert unter Zusatz von üblichen Umesterungskatalysatoren, beispielsweise einer aromatischen Sulfonsäure. Dabei braucht man nicht das Ende einer quantitativen Umesterung abzuwarten, sondern kann bereits bei einer teilweisen Umsetzung die Reaktion beenden. Zur weiteren Umsetzung nach dem erfindungsgemäßen Verfahren mit o-Phenylendiaminen müssen diese Umesterungskatalysatoren vom Umesterungsgemisch nicht abgetrennt werden. Sie können in der nachfolgenden Reaktion zur Herstellung von 2-(Cyanmethyl)benzimidazolen verbleiben, ohne jedoch hierbei Vorteile zu erbringen.

Die C₆-C₁₂-Alkylester ergeben zwar auch gute Ergebnisse, sind aber wegen des höheren Molekulargewichts bei einem prozentual kleineren Wirkstoffanteil 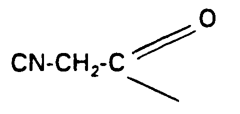
gewichtsmäßig unwirtschaftlich.

Als Lösemittel kommen für das erfindungsgemäße Verfahren alle halogenfreien Kohlenwasserstoffe, die auch Sauerstoffatome enthalten können, in Frage, die in Bezug auf die Reaktanden unter den gewählten Reaktionsbedingungen inert sind und einen Siedepunkt gleich oder höher als 150 °C haben. Die erfindungsgemäßen Lösemittel können Cycloaliphaten, Olefine, Cycloolefine, Alkoxyaromaten, Araliphaten oder Aromaten sein, vorzugsweise eine aromatische Kohlenwasserstofffraktion, dessen untere Grenze des Siedebereichs gleich oder höher als 150 °C ist, weiterhin Cumol, Phenetol, Mesitylen, Decalin, Tetralin, 1,2,3-Trimethylbenzol, 1,2,4-Trimethylbenzol, n-Propylbenzol, 1-Methyl-4-propylbenzol, 1-Methyl-4-ethylbenzol, 1-Methyl-3-ethylbenzol, 1-Methyl-2-ethylbenzol, o, m, p-Methyl-isopropylbenzol, 1,3-Diethylbenzol, 1,4-Diethylbenzol, 1-Ethyl-3,5-dimethylbenzol, 2-Ethyl-1,3-dimethylbenzol, 2-Ethyl-1,4-dimethylbenzol, 4-Ethyl-1,3-dimethylbenzol, 4-Ethyl-1,2-dimethylbenzol, 1-Methyl-2-propylbenzol, n-Butylbenzol, 1,2,4,5-Tetramethylbenzol, 1,2,3,5-Tetramethylbenzol, 1,2,3,4-Tetramethylbenzol oder Mischungen der genannten Verbindungen.

Als Lösemittel kommen insbesondere Cumol, Mesitylen, Tetralin, Phenetol oder ein Gemisch davon in Betracht.

Geeignet als Lösemittel sind ferner mono- und bicyclische Monoterpene mit oder ohne Sauerstoff, wie sie als technische Produkte aus natürlichen Rohstoffquellen, wie z.B. aus Terpentinölen oder durch Sulfataufschluß von Holz gewonnen werden können. [siehe Ullmann, 4. Auflage (1982) Band 22, Terpene, S. 535 ff und Terpentinöl, s.553 ff]. Bei diesen Produkten handelt es sich um ein Gemisch verschiedener Einzelkomponenten, die im wesentlichen Monoterpene sind. Als monocyclische Monoterpene kommen beispielsweise die Klassen der p-Menthadiene, wie α-Terpinen und Dipenten, und die bicyclischen Monoterpene, wie 3-Caren und α-Pinen, in Betracht. Als sauerstoffhaltige Monoterpene kommen beispielsweise α-Terpineol und Cineol in Betracht. Alle diese Lösemittel können in ökologisch günstiger Weise recycelt werden.

Die Umsetzung erfolgt im Temperaturbereich von 150 bis 175 °C, vorzugsweise 160 bis 170 °C. Während die Reaktion an der unteren Temperaturgrenze deutlich längere Reaktionszeiten benötigt, besteht an der oberen Temperaturgrenze schon die Gefahr der Bildung von erheblichen Mengen an Nebenprodukten. Besonders bevorzugt ist daher ein Temperaturbereich von 155 bis 170 °C. Je nach eingesetztem Cyanessigsäureester und Lösemittel gibt es für die besagte Kondensationsreaktion ein enges Temperaturoptimum, das von Fall zu Fall experimentell ermittelt werden muß.

Die stöchiometrischen Verhältnisse des eingesetzten o-Phenylendiamins und des Cyanessigsäureesters sind vorzugsweise 1:1,0 bis 1:1,1, insbesondere 1:1,04 bis 1:1,06. Ein Überschuß des o-Phenylendiamins ist nicht zweckmäßig. Die Ausbeute steigt nicht an und das überschüssige o-Phenylendiamin verunreinigt das Lösemittel. Ein geringer Überschuß des Cyanessigsäureesters von 4 bis 6 Mol-% ergibt die besten Ausbeuten, während ein größerer Überschuß nachteilig ist, da er die Ausbeute nicht steigert und entstehende Nebenprodukte das gewünschte Produkt verunreinigen.

Die Reaktionszeit beträgt zweckmäßigerweise 5 bis 30 Stunden. Während der Umsetzung werden abgespaltener Alkohol und abgespaltenes Wasser abdestilliert.

Nach dem erfindungsgemäßen Verfahren fallen die gewünschten Produkte in guter Reinheit und in kristalliner Form an, so daß sie in der Regel ohne zusätzliche Reinigung weiterverarbeitet werden können. Desweiteren kann auf ökologisch ungünstige Halogenbenzole wie o-Dichlorbenzol als Lösemittel verzichtet werden.

2-(Cyanmethyl)benzimidazol und seine Derivate sind wertvolle Zwischenprodukte für die Synthesen von Farbstoffen, Pigmenten, Pflanzenschutzmitteln und Pharmazeutika.

Zur Bestimmung der Reinheit wurden HPLC-Analysen durchgeführt. Die Proben wurden in Methanol im Ultraschallbad klar gelöst. Diese Lösung wurde über einen Vorfilter filtriert. Vorsäule und Hauptsäule waren RP-Säulen (reversed phase). Als Laufmittel dienten Methanol im Gemisch mit einem 0,5 %igen wäßrigen Natriumacetat/Eisessig-Puffer von pH 7. Die Eichung erfolgte gegen Reinsubstanz. 2-(Cyanmethyl)benzimidazol wurde bei 272 nm detektiert.

Die nachstehenden Beispiele dienen der Erläuterung der Erfindung. Die darin genannten Teile sind Gewichtsteile, die Prozentangaben stellen Gewichtsprozente dar, sofern nicht anders vermerkt.

### Beispiel 1

27 Teile 1,2-Diaminobenzol und 37 Teile Cyanessigsäure-iso-butylester wurden in 125 Teilen einer aromatischen Kohlenwasserstofffraktion mit einem Siedebereich von 185 - 211 °C 10 Stunden auf 165 °C unter einem Stickstoffstrom erhitzt. Entstandenes Isobutanol und Wasser wurden während dieser Zeit abdestilliert. Man ließ auf Raumtemperatur erkalten und saugte ab. Es wurde mit 40 Teilen der aromatischen Kohlenwasserstofffraktion gewaschen. Bei 100 bis 120 °C wurde im Vakuum das restliche Lösemittel aus dem Filterkuchen entfernt.

Auswaage: 35,4 Teile (90,2 % der Theorie) an gut kristallinem Produkt, Fp: 208 - 209 °C.
Eine Gehaltsbestimmung des Produkts mittels HPLC ergab einen Gehalt von 94,2 % an 2-(Cyanmethyl)benzimidazol. Die Ausbeute, bezogen auf 100 % iges Produkt, beträgt demnach 85 % d. Th.

### Vergleichsbeispiel 1

Vergleicht man vorstehendes Beispiel 1 mit Beispiel 2 der FR-A-1 361 778, in dem ebenfalls Cyanessigsäure-iso-butylester eingesetzt wird, so ergibt sich folgende Gegenüberstellung, aus der die Überlegenheit des erfindungsgemäßen Verfahrens hervorgeht:

| | Beispiel 1 | Vergleichsbeispiel Beispiel 2 aus FR-A-1 361 778 |
|---|---|---|
| Auswaage [%] | 90,2 | 70 |
| Fp. [°C] | 208 - 209 | 201 |
| Reinheit [%] | 94,2 | Angabe fehlt |
| Ausbeute [%] | 85 | < 70 |
| eingesetzter Katalysator | keiner | p-Toluolsulfonsäure |
| Reaktionstemperatur [°C] | 165 | 180 - 190 |
| Lösemittel | aromatischer Kohlenwasserstoff | o-Dichlorbenzol |

### Beispiel 2

27 Teile 1,2-Diaminobenzol und 29,5 Teile Cyanessigsäureethylester wurden in 125 Teilen einer aromatischen Kohlenwasserstofffraktion mit einem Siedebereich von 185 - 211 °C 10 Stunden auf 165 °C unter einem N₂-Strom erhitzt. Ethanol und Wasser wurden abdestilliert. Man ließ erkalten und wusch das Produkt mit 40 Teilen der aromatischen Kohlenwasserstofffraktion. Bei 100 - 120 °C wurde das restliche Lösemittel im Vakuum entfernt. Das nach diesem Beispiel hergestellte Produkt ist weniger gut kristallin als das in Beispiel 1.

Auswaage: 36,8 Teile Produkt.
Die HPLC-Bestimmung des Produkts ergab einen Gehalt von 91,4 % 2-(Cyanmethyl)benzimidazol. Die Ausbeute, bezogen auf 100 %iges Produkt, beträgt demnach 86 % d. Th.

### Vergleichsbeispiel 2

Vergleicht man die Ergebnisse des vorstehenden Beispiels 2 mit dem Beispiel 3 der indischen Patentschrift 154 773, in dem ebenfalls Cyanessigsäureethylester eingesetzt wird, so ergibt sich folgende Gegenüberstellung, aus der die Überlegenheit des erfindungsgemäßen Verfahrens hinsichtlich der Reinheit des Produktes, Ausbeute, gewähltem Lösemittel, Wirtschaftlichkeit und Entbehrlichkeit eines Katalysators hervorgeht:

| | Beispiel 2 | Beispiel 3 der indischen Patentschrift 154 773 | |
|---|---|---|---|
| | | Angaben | durch Nacharbeitung gefunden |
| Auswaage [%] | 93,8 | 90 | 98 |
| Reinheit [%] | 91,4 | fehlt | 77,5 |
| Ausbeute [%] | 85,6 | fehlt | 76 |
| eingesetzter Katalysator | keiner | p-Toluolsulfonsäure | |
| Lösemittel | aromatischer Kohlenwasserstoff | o-Dichlorbenzol | |
| Reaktionstemperatur | 165 °C | 175 °C | |
| Filtrationstemperatur | 25 °C | 0 - 5 °C | |

### Beispiel 3

27 Teile 1,2-Diaminobenzol und 37 Teile Cyanessigsäure-n-butylester wurden in 125 Teilen Cumol (Sp. 152 °C) 24 Stunden unter Stickstoffstrom unter Rückfluß erhitzt. Butanol und Wasser wurden abdestilliert. Man ließ erkalten und wusch das Produkt mit Cumol nach. Bei 100 - 120 °C wurde das restliche Lösemittel im Vakuum entfernt.
Auswaage: 84 % d.Th., Fp. 206 - 209 °C. Die HPLC-Bestimmung ergab einen Produktgehalt von 85,6 %. Die Ausbeute, bezogen auf 100 %iges Produkt, beträgt demnach 72 % der Theorie. Dieser Versuch zeigt, daß an der unteren erfindungsgemäßen Temperaturgrenze die Ausbeute bereits sinkt.

### Vergleichsbeispiel 3

Geht man über die erfindungsgemäße Temperaturgrenze von maximal 175 °C hinaus, beispielsweise auf 180 °C, so erhält man eine wesentlich schlechtere Ausbeute. Verfährt man wie in dem nachstehenden Beispiel 4a und erhitzt statt auf 167 °C auf 180 °C, so werden folgende Werte gefunden:
Auswaage: 42,0 g, Fp 192 - 250 °C (Zersetzung)
Die HPLC-Bestimmung ergab einen Produktgehalt von 33,8 %. Die Ausbeute, bezogen auf 100 %ige Ware, betrug demnach nur 36 % d. Th.

### Beispiel 4a

27 Teile 1,2-Diaminobenzol und 37 Teile Cyanessigsäure-n-butylester wurden in 125 Teilen einer aromatischen Kohlenwasserstofffraktion mit einem Siedebereich von 185 - 211 °C 10 Stunden auf 167 °C unter einem Stickstoffstrom erhitzt. n-Butanol und Wasser wurden abdestilliert. Man ließ erkalten und saugte ab. Es wurde mit 40 Teilen der aromatischen Kohlenwasserstofffraktion nachgewaschen. Bei 100 - 120 °C wurde im Vakuum das restliche Lösemittel entfernt. Das Produkt ist gut kristallin. Auswaage: 33,8 Teile an Produkt, Fp. 208 - 210 °C. Eine Gehaltsbestimmung des Produkts mittels HPLC ergab einen Gehalt an 2-(Cyanmethyl)benzimidazol von 95,1 %. Die Ausbeute, bezogen auf 100 %iges Produkt, beträgt demnach 86 % der Theorie.

### Beispiel 4b

Man führte den Versuch wie in Beispiel 4a durch, wobei man anstelle der dort verwendeten aromatischen Kohlenwasserstofffraktion eine tiefer siedende aromatische Kohlenwasserstofffraktion mit einem Siedebereich von 166 - 184 °C verwendete, und erhielt folgendes Ergebnis:
Auswaage: 36,6 Teile Produkt, Fp.: 203 - 207 °C.

Eine Gehaltsbestimmung des Produkts mittels HPLC ergab einen Gehalt an 2-(Cyanmethyl)benzimidazol von 94,2 %.
Die Ausbeute, bezogen auf 100% iges Produkt, beträgt demnach 87,8 % der Theorie.

### Beispiele 5 bis 10

Man verfuhr analog Beispiel 4a und setzte die in der nachstehenden Tabelle angegebenen Cyanessigsäureester bei den genannten Reaktionstemperaturen ein.

| Beispiel | Cyanessigsäureester (R² =) | Reaktionstemperatur [°C] | Schmelzpunkte des erhaltenen Produkts [°C] | Reinheit [%] | Ausbeute (auf 100 %iges Produkt bezogen) [%] |
|---|---|---|---|---|---|
| 5 | Propyl | 160 | 207 - 209 | 96,8 | 83 |
| 6 | Pentyl | 165 | 209 - 210 | 99,9 | 85 |
| 7 | 2-Ethylhexyl | 167 | 208 - 211 | 98,5 | 85 |
| 8 | n-Octyl | 167 | 208 - 210 | 99,5 | 80 |
| 9 | 2-Methoxyethyl | 160 | 201 - 207 | 91,6 | 85 |
| 10 | 2-Ethoxyethyl | 158 | 208 - 210 | 99,8 | 86 |

### Beispiele 11a - c

Man verfuhr wie in Beispiel 2 und ersetzte den Cyanessigsäureethylester jeweils zu a: 75 %, b: 50 %, c: 25 % durch Cyanessigsäure-n-butylester und erhielt Produkte guter Kristallinität mit folgenden Schmelzpunkten und Ausbeuten:

| Beispiel | Fp [°C] | Ausbeute [% d. Th.] bezogen auf 100% iges Produkt |
|---|---|---|
| 11a | 202 - 210 | 86 |
| 11b | 204 - 209 | 87 |
| 11c | 203 - 208 | 88 |

### Beispiel 12

27 Teile 1,2-Diaminobenzol und 37 Teile Cyanessigsäure-n-butylester wurden in 125 Teilen Phenetol 10 Stunden auf 165 - 166 °C unter einem Stickstoffstrom erhitzt. n-Butanol und Wasser wurden abdestilliert. Man ließ auf Raumtemperatur erkalten und saugte ab. Es wurde mit 40 Teilen Phenetol nachgewaschen. Der Filterkuchen wurde im Vakuum bei 100 - 120 °C getrocknet. Man erhielt ein gut kristallines Produkt.

Auswaage: 34,4 Teile, Fp. 205 - 210 °C. Eine Gehaltsbestimmung mittels HPLC ergab einen Produktgehalt von 96,7 %. Die Ausbeute, bezogen auf 100 %iges Produkt, betrug demnach 84,8 % der Theorie.

### Beispiel 13

Man verfuhr wie in Beispiel 12 und verwendete anstelle von Phenetol ein technisches Gemisch aus p-Menthadienen und erhielt folgende Ergebnisse:

Auswaage: 36,6 Teile, Fp. 202 - 207 °C.
Eine Gehaltsbestimmung mittels HPLC ergab einen Produktgehalt von 89,6 %. Die Ausbeute, bezogen auf 100 %iges Produkt, betrug demnach 83,5 % d. Th..

### Beispiel 14

### 5-Methyl-2-(cyanmethyl)benzimidazol

30,5 Teile 3,4-Diaminotoluol und 37,0 Teile Cyanessigsäure-n-butylester wurden unter einem Stickstoffstrom in 125 Teilen einer aromatischen Kohlenwasserstofffraktion mit einem Siedebereich von 185 - 211 °C 10 Stunden auf 160 °C erhitzt, wobei das abgespaltene n-Butanol und Wasser abdestilliert wurden. Man ließ erkalten und filtrierte ab. Es wurde mit 45 Teilen der aromatischen Kohlenwasserstofffraktion gewaschen. Bei 100 °C wurde dem Produkt im Vakuum das restliche anhaftende Lösemittel entzogen.
Auswaage: 34 Teile des Produkts, 80 % d. Th.
Fp.: 186 - 187 °C.

Dieses Verfahren ist dem von J. Sawlewicz et al. in Pol. J. Pharmacol. Pharm. 1974, 26, S. 642 beschriebenen Verfahren zur Herstellung von 5-Methyl-2-(cyanmethyl)benzimidazol mit einer Ausbeute von 44 % bei praktisch gleichem Schmelzpunkt (186 - 188 °C) deutlich überlegen.

## Patentansprüche

1. Verfahren zur Herstellung von substituiertem oder unsubstituiertem 2-(Cyanmethyl)benzimidazol der Formel (I), 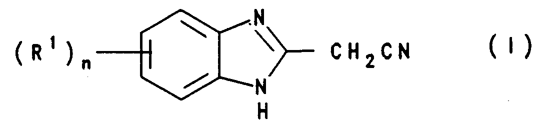 dadurch gekennzeichnet, daß man ein o-Phenylendiamin der Formel (II) 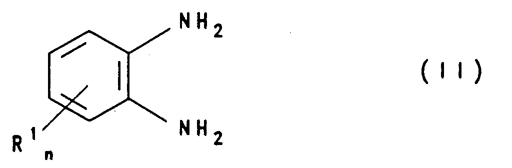 wobei R¹ C₁-C₄-Alkyl oder C₁-C₄-Alkoxy bedeutet und n eine ganze Zahl von 0 bis 2 ist,
mit einem Cyanessigsäureester der Formel (III),
NC-CH₂-COOR² (III)
wobei R² lineares, verzweigtes oder cyclisches C₂-C₁₂-Alkyl bedeutet und 1 bis 3 CH₂-Gruppen des Alkylrestes jeweils durch Sauerstoff ersetzt sein können, bei einer Temperatur von 150 - 175 °C, in einem halogenfreien, in Bezug auf die Reaktanden unter den gewählten Reaktionsbedingungen inerten Lösemittel, das einen Siedepunkt gleich oder höher als 150°C hat und aus der Gruppe der Cycloaliphaten, Olefine, Cycloolefine, Aromaten oder Araliphaten ausgewählt ist, wobei besagte Lösemittel auch Sauerstoffatome enthalten können, oder in einem Gemisch aus zwei oder mehreren solcher Lösemittel, umsetzt, ohne eine aromatische Sulfonsäure als Katalysator zuzusetzen.

2. Verfahren nach Anspruch 1, dadurch gekennzeichnet, daß die Umsetzung bei einer Temperatur von 155 bis 170 °C erfolgt.

3. Verfahren nach Anspruch 1 oder 2, dadurch gekennzeichnet, daß n die Zahl 1 ist.

4. Verfahren nach mindestens einem der Ansprüche 1 bis 3, dadurch gekennzeichnet, daß R¹ die Bedeutung Methyl oder Methoxy hat.

5. Verfahren nach Anspruch 1 oder 2, dadurch gekennzeichnet, daß n die Zahl 0 ist.

6. Verfahren nach mindestens einem der Ansprüche 1 bis 5, dadurch gekennzeichnet, daß R² Ethyl, n-Propyl, n-Butyl, iso-Butyl, n-Pentyl, iso-Pentyl, 2-Ethylhexyl, n-Octyl, 2-Methoxyethyl oder 2-Ethoxyethyl bedeutet.

7. Verfahren nach mindestens einem der Ansprüche 1 bis 5, dadurch gekennzeichnet, daß ein Gemisch verschiedener Cyanessigsäureester der Formel (III) eingesetzt wird.

8. Verfahren nach mindestens einem der Ansprüche 1 bis 7, dadurch gekennzeichnet, daß Cyanessigsäureethylester im Gemisch mit Cyanessigsäure-n-butylester oder mit Cyanessigsäure-iso-butylester eingesetzt wird.

9. Verfahren nach mindestens einem der Ansprüche 1 bis 8, dadurch gekennzeichnet, daß das Lösemittel ein Aromat, ein Araliphat oder Alkoxyaromat mit einem Siedepunkt gleich oder höher als 150 °C oder eine aromatische Kohlenwasserstofffraktion ist, deren untere Grenze des Siedebereichs gleich oder höher als 150 °C ist.

10. Verfahren nach mindestens einem der Ansprüche 1 bis 8, dadurch gekennzeichnet, daß das Lösemittel Cumol, Mesitylen, Tetralin, Phenetol oder ein Gemisch davon ist.

11. Verfahren nach mindestens einem der Ansprüche 1 bis 8, dadurch gekennzeichnet, daß das Lösemittel ein monocyclisches oder bicyclisches Monoterpen oder ein Gemisch ist, welches im wesentlichen aus Monoterpenen besteht.

## Claims

1. A process for the preparation of substituted or unsubstituted 2-(cyanomethyl)benzimidazole of the formula (I) 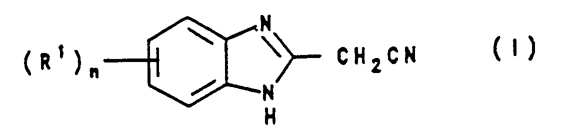 which comprises reacting an o-phenylenediamine of the formula (II) 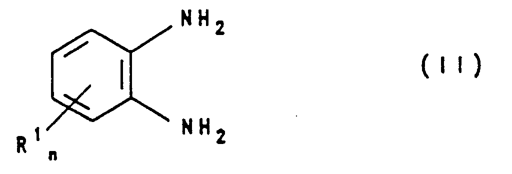 in which R¹ is C₁-C₄-alkyl or C₁-C₄-alkoxy and n is an integer from 0 to 2,
with a cyanoacetic ester of the formula (III)
NC-CH₂-COOR² (III)
in which R² is linear, branched or cyclic C₂-C₁₂-alkyl and 1 to 3 CH₂ groups of the alkyl radical can in each case be replaced by oxygen, at a temperature of 150 - 175°C, in a halogen-free solvent which is inert as regards the reactants under the selected reaction conditions, has a boiling point of 150°C or above and is selected from the group consisting of cycloaliphatic substances, olefins, cycloolefins, or aromatic or araliphatic substances, it also being possible for said solvent to contain oxygen atoms, or in a mixture of two or more of such solvents, without adding an aromatic sulfonic acid as catalyst.

2. The process as claimed in claim 1, wherein the reaction is carried out at a temperature of 155 to 170°C.

3. The process as claimed in claim 1 or 2, wherein n is the number 1.

4. The process as claimed in at least one of claims 1 to 3, wherein R¹ is methyl or methoxy.

5. The process as claimed in claim 1 or 2, wherein n is the number 0.

6. The process as claimed in at least one of claims 1 to 5, wherein R² is ethyl, n-propyl, n-butyl, iso-butyl, n-pentyl, iso-pentyl, 2-ethylhexyl, n-octyl, 2-methoxyethyl or 2-ethoxyethyl.

7. The process as claimed in at least one of claims 1 to 5, wherein a mixture of various cyanoacetic esters of the formula (III) is employed.

8. The process as claimed in at least one of claims 1 to 7, wherein ethyl cyanoacetate is employed in a mixture with n-butyl cyanoacetate or with iso-butyl cyanoacetate.

9. The process as claimed in at least one of claims 1 to 8, wherein the solvent is an aromatic, araliphatic or alkoxyaromatic substance whose boiling point is 150°C or above or an aromatic hydrocarbon fraction whose lower limit of the boiling range is 150°C or above.

10. The process as claimed in at least one of claims 1 to 8, wherein the solvent is cumene, mesitylene, tetralin, phenetole or a mixture thereof.

11. The process as claimed in at least one of claims 1 to 8, wherein the solvent is a monocyclic or bicyclic monoterpene or a mixture which is composed essentially of monoterpenes.

## Revendications

1. Procédé pour la préparation de 2-(cyanométhyl)benzimidazole substitué ou non substitué de formule (I) : 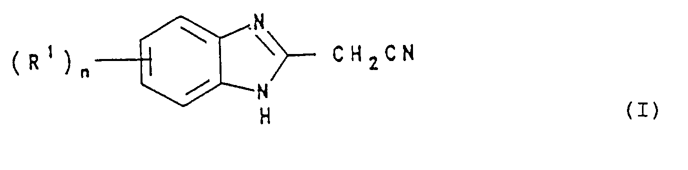 caractérisé en ce que l'on fait réagir une o-phénylènediamine de formule (II) : 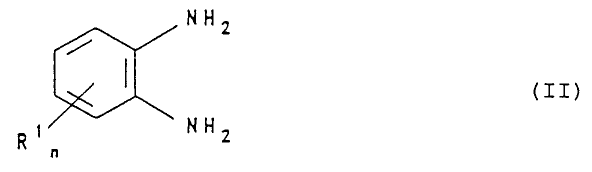 R¹ étant alkyle en C₁-C₄ ou alcoxy en C₁-C₄, et n un nombre entier de 0 à 2,
avec un ester d'acide cyanoacétique de formule (III) :
NC-CH₂-COOR² (III)
où R² représente un alkyle en C₂-C₁₂ linéaire, ramifié ou cyclique et 1 à 3 groupes CH₂ du radical alkyle pouvant être chacun remplacé par l'oxygène, à une température de 150 à 175 °C, dans un solvant non halogéné inerte vis-à-vis des réactants dans des conditions de réaction choisies, qui a un point d'ébullition égal ou supérieur à 150 °C, et que l'on choisit dans un groupe comportant des cycloaliphatiques, des oléfines, des cyclooléfines, des aromatiques ou araliphatiques, les solvants cités pouvant contenir également des atomes d'oxygène, ou un mélange de deux ou plusieurs de tels solvants, sans addition d'un acide sulfonique aromatique comme catalyseur.

2. Procédé selon la revendication 1, caractérisé en ce que l'on met en oeuvre la réaction à une température de 155 à 170 °C.

3. Procédé selon la revendication 1 ou 2, caractérisé en ce que n vaut 1.

4. Procédé selon au moins l'une des revendications 1 à 3, caractérisé en ce que R¹ représente méthyle ou méthoxy.

5. Procédé selon la revendication 1 ou 2, caractérisé en ce que n vaut 0.

6. Procédé selon au moins l'une des revendications 1 à 5, caractérisé en ce que R² représente éthyle, n-propyle, n-butyle, iso-butyle, n-pentyle, iso-pentyle, 2-éthylhexyle, n-octyle, 2-méthoxyéthyle ou 2-éthoxyéthyle.

7. Procédé selon au moins l'une des revendications 1 à 5, caractérisé en ce que l'on utilise un mélange de différents cyanoacétates de formule (III).

8. Procédé selon au moins l'une des revendications 1 à 7, caractérisé en ce que l'on utilise le cyanoacétate d'éthyle en mélange avec le cyanoacétate de n-butyle ou le cyanoacétate d'isobutyle.

9. Procédé selon au moins l'une des revendications 1 à 8, caractérisé en ce que l'on utilise en tant que solvant un aromatique, un araliphatique ou un alcoxyaromatique ayant un point d'ébullition identique ou supérieur à 150 °C, ou une fraction hydrocarbonée aromatique dont la limite inférieure du domaine d'ébullition est égal ou supérieure à 150 °C.

10. Procédé selon au moins l'une des revendications 1 à 8, caractérisé en ce que l'on utilise en tant que solvant le cumène, le mésitylène, la tétraline, le phénétol ou un mélange de ces solvants.

11. Procédé selon au moins l'une des revendications 1 à 8, caractérisé en ce que le solvant est un monoterpène monocyclique ou bicyclique ou un mélange qui se compose essentiellement de monoterpènes.
